# EUROPÄISCHE PATENTSCHRIFT

(11) **EP 1 830 836 B1**
(45) Veröffentlichungstag und Bekanntmachung des Hinweises auf die Patenterteilung: **24.11.2010**
(21) Anmeldenummer: 04822593.2
(22) Anmeldetag: 22.12.2004
(51) Int. Cl.: A61K 31/357, A61K 31/165, A61P 23/00

(54) **MISCHUNG EINES VANILLOIDREZEPTORAGONISTEN MIT EINER NERVENREGENERATIONSHEMMENDEN SUBSTANZ, IHRE VERWENDUNG FÜR DIE HERSTELLUNG EINES SCHMERZMITTELS UND VERFAHREN ZUR APPLIKATION DIESES MITTELS**
MIXTURE OF A VANILLOID RECEPTOR AGONIST AND A SUBSTANCE INHIBITING NERVE REGENERATION, USE THEREOF FOR PRODUCING A PAINKILLER, AND METHOD FOR APPLYING SAID PAINKILLER
MELANGE CONTENANT UN AGONISTE DES RECEPTEURS VANILLOIDES ET UNE SUBSTANCE INHIBANT LA REGENERATION DES NERFS, UTILISATION DE CE MELANGE POUR PRODUIRE UN ANALGESIQUE ET PROCEDE D'APPLICATION DE CET ANALGESIQUE

(43) Veröffentlichungstag der Anmeldung: 12.09.2007
(73) Patentinhaber: Mestex AG, 8008 Zürich (CH)
(72) Erfinder: MEYER, Dominik, CH-8008 Zürich (CH)
(74) Vertreter: Lusuardi, Werther
(86) Internationale Anmeldenummer: PCT/CH2004/000750
(87) Internationale Veröffentlichungsnummer: WO 2006/066419

(56) Entgegenhaltungen:
- WO-A-98/51290
- WO-A-03/015698
- WO-A-2004/056305
- WO-A-2004/058286
- US-A1- 2003 082 249
- KARAI L ET AL: "Deletion of vanilloid receptor 1-expressing primary afferent neurons for pain control" JOURNAL OF CLINICAL INVESTIGATION 2004 UNITED STATES, Bd. 113, Nr. 9, 2004, Seiten 1344-1352, XP002333034 ISSN: 0021-9738

## Beschreibung

Die Erfindung bezieht sich auf die Mischung eines Vanilloidrezeptoragonisten (untenstehend auch "Vanilloide" genannt) mit einer nervenregenerationshemmenden Substanz (untenstehend auch "Inhibitor" genannt) und ihre Verwendung zur Herstellung eines Mittels (untenstehend auch "Substanzkombination" zur Behandlung von Schmerzen.
Solche Mittel können systemisch, regional, topisch oder lokal eingenommen werden.

Die erfindungsgemässe Mischung eignet sich zur Verlängerung der Wirkung von Vanilloidrezeptoragonisten insbesondere für den Vanilloidrezeptor Typ I (z.B. Resiniferatoxin) zur dauerhaften Therapie jeglicher Form von Schmerzen, welche von nociceptiven Fasern weitergeleitet werden, insbesondere auch neurogene Schmerzen. Ferner kann die Substanzkombination dazu dienen jegliche andere Wirkung von Vanilloidrezeptoragonisten wie Thermoregulation oder andere bekannte Effekte zu verlängern durch Hemmung der Regeneration der betroffenen Nerven.

Exemplarisch wird unten die Verwendung dieser Mischung vor allem anhand der Behandlung von Gelenkschmerzen ausgeführt, sie soll aber für jegliche Form von Schmerzen gelten. Von Gelenken ausgehende Schmerzen haben ihren Ursprung häufig im Bereich der Gelenkkapsel oder im gelenknahen Bereich des Knochens. Dabei können viele Ätiologien in Frage kommen, z.B. arthrotische oder arthritische Krankheitsformen, mechanische oder andere Reizung der gelenknahen Knochenoberfläche, Reizung oder Verletzung der Gelenksbandstrukturen, Infekte, autoimmune Prozesse, u.s.w. In allen Fällen, welche im Rahmen dieser Erfindung von Interesse sind, gehen die entstehenden Schmerzen von nociceptiven Nervenfasern im gelenknahen Bereich aus. Nociceptive Nervenfasern werden auch als C-Fasern und A-delta Fasern bezeichnet. Wird in ein so erkranktes Gelenk eine analgetische Substanz (z.B. Lokalanästhetika, Vanilloidrezeptoragonisten oder Morphine) injiziert, so werden die Beschwerden des Patienten gelindert. Allerdings haben die heute gebräuchlichen Substanzen eine nur beschränkte Wirkdauer, weshalb die Beschwerden meistens zurückkehren.

Zur Therapie schmerzhaft erkrankter Gelenke werden heute generell folgende Verfahren angewendet:
- Physiotherapie/Bewegungstherapie
- Systemische analgetische/antiphlogistische Therapie (etc.)
- Lokale analgetischelantiphlogistische Verfahren (etc.)
- Operative Verfahren:
- Arthroskopisch: Debridement, Gelenkstoilette, etc.
- Offen/Mini-offen: Gelenksersatz, Gelenkversteifung, etc.

In der Literatur wurden auch schon eine Reihe von bekannten Substanzen zur Therapie schmerzhafter, entzündlicher Gelenke vorgeschlagen, insbesondere:
- Injektion von Capsaicinen
- Osmiumsäure oder radioaktive Substanzen wie Technetium 99, welche zu einer Synoviorthese führen
- Injektion von Lokalanästhetika, Hyaluronsäurepräparaten (etc.)
- Injektion von Antiphlogistika
- Injektion von Kontrastmitteln zur Gelenksdiagnostik
- Gelenkspülung zur Gelenkstoilette
- Gelenkferne chemische, thermische, elektrische oder chirurgische Ablation der gelenksversorgenden Nerven.

So besteht beispielsweise beim bekannten Verfahren der Synoviorthese der Nachteil der Zerstörung der molekularen Strukturen, insbesondere Denaturierung der Proteine, welche im Prozess der Arthritis und z.T. auch Arthroseentwicklung als Entzündungsauslöser wirken. Dabei entsteht eine Fibrose der Gelenkkapsel welche weniger entzündlich und somit auch weniger schmerzhaft ist. Gleichzeitig wird durch die bei der Synoviorthese auftretende Fibrose des Gelenkes die zumeist vorhandene und dabei ebenfalls zu behandelnde Hyperämie vermindert, woraus sich ebenfalls therapeutischer Nutzen ergibt. Die fibrotische Vernarbung nach Synoviorthese kann aber zu einer verminderten Beweglichkeit des Gelenkes führen, sowie zu einer verminderten Produktion von Synovialflüssigkeit und zur Zerstörung des Gelenkknorpels. Diese unerwünschte Fibrose der Gelenkskapsel sollte vermieden und nur die sensible Innervation des Gelenkes ausgeschaltet werden.

Aus der EP-B 0 998 288 CAMPBELL ist die Verwendung von Capsaicin und Analogen davon zusammen mit einem Lokalanästhetikum bekannt. Capsaicin hat eine stark brennende Sensation zur Folge, so dass diese nur durch eine simultane oder sequentielle Verabreichung eines Lokalanästhetikums gelindert werden kann. Lokalanästhetika haben eine antagonistische Wirkung zu Capsaicin, welche die Wirksamkeit des Capsaicins herabsetzen, dies wiederum führt dazu, dass eine grössere Dosierung bzw. eine höhere Konzentration von Capsaicin notwendig ist, wenn diese zusammen mit einem Lokalanästhetikum verwendet wird. In der so benötigten Dosierung hat Capsaicin den Effekt, dass eine entzündliche Reaktion verursacht wird und bei Anwendung im Gelenk ein Knorpelschaden verursacht wird. Eine kombinierte Verwendung der hier beanspruchten Substanzkombination mit Lokalanästhetika zur Reduktion der Schmerzen bei Injektion ist aber durchaus möglich.

Aus der US 6,326,020 KOHANE TE AL. ist die Verwendung eines Vanilloidrezeptoragonisten zusammen mit einem Natrium-Kanal-Blocker vom Typ von Tetrodotoxin bekannt "site 1 sodium channel blocker" zur Erzielung einer Nervenblockade bekannt. Diese Klasse von Substanzen hat jedoch lediglich eine zusätzliche toxische Wirkung auf die Nerven, nicht aber eine wirkungsverlängernde Wirkung im Sinne dieser Erfindung.

Aus der wissenschaftlichen Publikation "Deletion of vanilloid receptor 1-expressing primary afferent neurons for pain control" der Autoren Karai et al., in The Journal of Clinical Investigations, vol. 113, No. 9, May 2004 wird die Verwendung von Resiniferatoxin zur selektiven Zerstörung von D-Fasern offenbart, um eine anhaltende Schmerzlinderung bei chronischen Schmerzen zu erzielen.

Die WO 2004/056305 BURCH beschreibt die histologischen Vorgänge nach Applikation von Resiniferatoxin als axonale Waller-Degeneration und offenbart als einen hieraus folgenden Nachteil der Methode, dass die Wirkdauer durch Regeneration des Axons begrenzt wird.

Alle bisher verwendeten Substanzen und Verfahren führen nur zu einer relativ kurzfristigen oder unvollständigen Schmerzfreiheit oder verursachen bleibende Schädigungen am Gelenk.

Hier will die Erfindung Abhilfe schaffen. Durch die Mischung des Vanilloidrezeptoragonisten mit gewissen nervenregenerationshemmenden Substanzen kann die notwendige Dosierung des neurotoxischen Vanilloidrezeptoragonisten deutlich reduziert werden und damit auch die Nebenwirkungen wie ein schmerzhaftes Brennen bei Injektion, lokale Entzündung sowie Schäden am Gewebe wie z.B: Knorpelschaden am Gelenk. Bei Injektion bei einem sensiblen Patienten oder an einer schmerzhaften Stelle kann fakultativ vor, mit oder nach Injektion der hier beanspruchten Substanzen eine lokal-anästhetische Substanz, z.B. Lidocain, injiziert werden (gemäss Campbell et al.). Ein Weg diese Symptome und Nebenwirkungen weiter zu reduzieren sowie die Effizienz der Therapie zu steigern besteht darin, eine oder beide oder mehrere der Wirksubstanzen durch geeignete galenische Formulierungen verzögert freizusetzen.

Die Erfindung löst die gestellte Aufgabe mit einer Mischung gemäss den Merkmalen des Anspruchs 1 sowie der Verwendung einen solchen Mischung gemäss den Merkmalen des Anspruchs 23.

Die Wirkung von Vanilloidrezeptoragonisten (als Schmerzmittel, lokal oder systemisch, topisch oder auf irgendeine Weise in den Körper gebracht) ist reversibel durch Heilung der Nerven, insbesondere der C- und A-delta Fasern. Durch Zugabe von nervenregenerationshemmenden Substanzen (lokal oder systemisch, topisch oder im Körper) wird die Wirkung des Schmerzmittels überraschenderweise verstärkt und / oder verlängert.

Erfindungswesentlich ist die Erkenntnis, dass Vanilloidrezeptoragonisten die rezeptortragenden Nerven schädigen, so dass diese keine Reizantwort mehr weiterleiten können. Dadurch wird der selektiv geschädigte Nerv (z.B. eine C-Faser oder eine C-Faser-Endigung) empfindlicher für die nervenregenerationshemmenden Substanzen, welche z.B. durch Blockade des tubulären und oder axonalen Transportsystems, durch Tubulusaggregation oder Aggregationshemmung, Tubuluspolymerisation oder Tubulusdepolymerisation, Tubulusschädigung durch andere die Neurotubuli oder Tubulin beeinträchtigenden Mechanismen, oder durch tubulären oder axonalen retrograden "Suizidtransport" die Nervenregeneration hemmen und die Nervenhomöostase beeinträchtigen. Dadurch wird nicht nur die Nervenregeneration gehemmt, sondern der Nerv auch teilweise abgetötet und ein Abbau des Nerven induziert, wodurch kein Nachwachsen, Neuwachsen, Regeneration oder Aussprossen mehr möglich ist oder dieses deutlich reduziert wird.

Ein wesentlicher Aspekt der Erfindung ist, dass die ermittelten nervenregenerationshemmenden Substanzen überraschenderweise bereits bei einer sehr geringgradigen Schädigung der sensiblen Nervenfaser durch den Vanilloidrezeptoragonisten in den Nerven eindringen und so eine Wirkung im Sinne des Vanilloidrezeptoragonisten hervorrufen können, in einer Dosierung oder Konzentration welche für beide Substanzen alleine nicht den gewünschten Effekt hätte. Durch die selektive Wirkung des Vanilloidrezeptoragonisten wird der Nerv so geschädigt, dass die regenerationshemmende Substanz bereits ihre Wirkung entfalten kann, während andere, nicht für Vanilloide sensible Fasern geschont bleiben. Daher ist es besonders effizient, beide Substanzen als Gemisch zu applizieren oder zu injizieren, da auf diese Weise sowohl örtlich dieselben Nerven geschädigt werden können als auch zeitlich die Substanzen die zu behandelnden Nerven und Nervenendigungen oder auch Ganglien oder Nervenzellen gleichzeitig in gewünschter Weise schädigen können. Diese Therapie ist in erster Linie für die Schmerztherapie entwickelt worden. Da nicht alle nervenregenerationshemmenden Substanzen an demselben Ort an den neuronalen Tubuli angreifen hat es sich als vorteilhaft erwiesen zwei oder mehrere verschiedene Tubulus-Gifte, z.B. Taxol mit Vincristin zu mischen. Ferner kann ein noch teilweise funktioneller Nerv keine neuen empfindlichen Rezeptoren an die Nervenendigung transportieren und bleibt so unsensibel.

Wie bereits oben erwähnt ist auch die Kombination beider (oder mehrerer) Substanzen systemisch, nur einer Substanz systemisch und die andere Substanz lokal (auch topisch auf Haut oder Schleimhaut) oder regional sowie beider (oder mehrerer) Substanzen lokal (auch topisch auf Haut oder Schleimhaut) oder regional denkbar, gleichzeitig oder zeitlich versetzt (in diesem Falle sollte vorzugsweise zuerst der Vanilloidrezeptoragonist, dann die regenerationshemmende Substanz verwendet werden) oder verzögert appliziert werden. Ferner ist die Zubereitung einer oder beider oder mehrere der Komponenten der Mischung mit/als zeitlich verzögerte oder gesteuert freisetzenden pharmakologischen Zubereitungen oder Trägermedien möglich und sinnvoll.

Die mit der Erfindung erzielbaren Vorteile sind die folgenden:
• Die lokale Anwendung von Vanilloidrezeptoragonisten und/oder Inhibitoren führt zu einer hauptsächlich lokalen Wirkung erlaubt die systemischen und regionalen Nebenwirkungen zu reduzieren, insbesondere auch die benötigte Menge der Substanzen.
• Die Kombination der beiden Substanzen wirkt synergistisch und verbessert Effizienz und Wirkungsdauer beider Einzelsubstanzen.
• Die lokale Anwendung von Vanilloidrezeptoragonisten kann die zu behandelnden Nerven lokal erreichen, die systemische Verabreichung der regenerationshemmenden Substanz erreicht, dass der entsprechende Nerv auf seiner gesamten Länge der Hemmung ausgesetzt wird was sich günstig auf die Wirkungsverlängerung und -Verstärkung auswirken kann. Durch die systemische Anwendung können lokale Nebenwirkungen verringert werden.
• Die systemische Anwendung von Vanilloidrezeptoragonisten und Analoga, insbesondere neueren, oral anwendbaren Substanzen, hat den Vorteil dass keine gezielte Applikation notwendig ist und lokale Nebenwirkungen reduziert, sowie die betroffenen Nervenstrukturen auf ihrer gesamten Länge erreicht werden können. Die zusätzliche lokale Inhibition mittels regenerationshemmenden Substanzen verhindert die lokale Regeneration.
• Die systemische Anwendung von Vanilloidrezeptoragonisten und Analoga, insbesondere neueren, oral anwendbaren Substanzen zusammen mit regenerationshemmenden Substanzen hat den Vorteil, dass keine gezielte Applikation notwendig ist und lokale Nebenwirkungen reduziert werden. Sämtliche Substanzen können aber auch zusammen oder getrennt intravenös, intra-arteriell, intraperitoneal, subcutan percutan (auch Schleimhäute und Urothel), peroral, inhaliert oder ähnlich verabreicht werden. Der Vorteil der systemischen Anwendung ist, dass die betroffenen Nervenstrukturen auf ihrer gesamten Länge erreicht werden können und mehrere Schmerzlokalisationen gleichzeitig behandelt werden können. Die zusätzliche lokale Inhibition mittels regenerationshemmenden Substanzen kann fakultativ verabreicht werden und verhindert die lokale Regeneration, alternativ oder zusätzlich kann auch lokal ein Vanilloidrezeptoragonist verwendet werden.
• Das Verfahren ist durch Nicht-Spezialisten durchführbar.
• Das Verfahren ist mit einer dünnen, auch nicht-arthroskopischen Nadel durchführbar.
• Das Verfahren ist nicht infektionsgefährdend, im Gegensatz zum beliebten Verfahren der Cortisoninjektion, welches stark lokal infektionsfördernd ist, da Cortison lokal das Immunsystem hemmt.
• Das Verfahren führt zu einer sensiblen Denervation, d.h. einer Ausschaltung von schmerzleitenden Nerven.
• Erweiterung der Gelenksbeweglichkeit durch Aufhebung der schmerzhaften Bewegungseinschränkung im Gegensatz zur Synoviorthose, bei welcher durch die entstehende Kapselfibrose eine Bewegungseinschränkung erfolgt.
• Positive Vorbereitung für eine spätere Arthroplastik bei Anwendung an einem Gelenk. Durch die stimulierende Wirkung der wieder stattfindenden Belastung ohne Schmerz wird die Knochenbildung in der Extremität insbesondere in Gelenknähe gefördert und der gelenknahe Knochen erhält eine für den späteren Halt einer Prothese vorteilhaftere Struktur.
• Keine lokale Fettgeweberesorption (Lipolyse)
• Keine Schwächung von kollagenen Sehnen/Band/Kapsel-Strukturen.
• Durch die hochselektive Wirkung von Vanilloidrezeptoragonisten tritt keine motorische Beeinträchtigung ein, egal wo die Substanz gespritzt wird.
• Durch die hochselektive Wirkung von Vanilloidrezeptoragonisten tritt keine proprioceptive Beeinträchtigung ein, egal wo die Substanz gespritzt wird.

Die nervenregenerationshemmenden Substanzen gemäss der Gruppe a) des Patentanspruchs 1 sind besonders effizient in der Schädigung der Nervenregeneration. Die nervenregenerationshemmenden Substanzen gemäss der Gruppe b) des Patentanspruchs 1. Die nervenregenerationshemmenden Substanzen gemäss der Gruppe c) haben sehr wenige Nebenwirkungen ausser einer z.T. vergrösserten Blutungsneigung.

Die Erfindung wird im Folgenden für die Anwendung beim Menschen beschrieben, die angegebenen Dosierungen beziehen sich somit auf die Humanapplikation. Die Erfindung eignet sich aber auch für den Veterinärbereich oder im experimentellen Rahmen im Labor, wobei dort Anpassungen in der Dosierung vorgenommen werden müssen in Abhängigkeit vom Körpergewicht des jeweiligen Tieres.

### Intraartikuläre Anwendung:

Bei dieser Anwendung wird die Gelenkskapsel oder der Gelenkschmierbeutel dazu verwendet die Wirkung der beanspruchten Substanz-Kombination auf den Ort der Schmerzentstehung zu konzentrieren und dadurch lokal eine höhere Konzentration beider oder einer der Komponenten zu erlauben, als es ohne die schützende Gelenkskapsel oder den Gelenkschmierbeutel in der gleichen Konzentration und Verträglichkeit möglich wäre und gleichzeitig die Gefäss-/Nervenstrukturen und andere Strukturen in der Nähe des Gelenkes verhältnismässig zu schonen. Somit wird eine langfristige Linderung der vom dem erkrankten Band-Kapsel-Gelenk-Komplex ausgehenden Schmerzempfindung durch Hemmung oder Ausschaltung der Reizleitung erlangt. Dieses Verfahren kann sowohl präventiv oder therapeutisch angewandt werden.
Bei einer bevorzugten Ausführungsform wird zusätzlich zur Substanzkombination ein Röntgenkontrastmittel, z.B. ein Bariumzusatz oder ein MRI-Kontrastmittel verwendet, so dass eine bildgebende Kontrolle der Verteilung Der Substanzkombination im intrakapsulären Raum möglich ist.
Als Kontrastmittel können je nach Verfahren folgende Substanzen verwendet werden:

| | |
|---|---|
| Röntgen, CT: | Jodhaltige Substanzen, z.B. trijodierte Benzoate oder lopamidol, |
| | idealerweise 30 - 80g/100ml oder |
| | z. B. 5 - 10% eines anderen Kontrastmittels, z.B. Barium. |
| MRI: | z.B. Gadolinium, z.B. pro 1ml: 469,01 mg Gadopentat Dimeglumid, 0,99 mg Meglumin, 0,4 mg Diethylentriamin-pentaacetat. |

Bei einer weiteren Ausführungsform wird zusätzlich zur Substanzkombination eine antibiotische, desinfizierende und/oder sterilisierende Substanz beigefügt.
Bei einer weiteren Ausführungsform wird zusätzlich zur Substanzkombination ein visköser Zusatzstoff, z.B. Glycosaminoglycan, Chondroitinsulfat und / oder Hyaluronsäure und vergleichbare Substanzen, vorzugsweise mit einer Konzentration von 0,1-50,0 mg/ml Injektionslösung verwendet, was zu einer mechanischen Gleitverbesserung des Gelenkes führt und Schmerzen bei Injektion vermindert. Ferner ist eine Wirkungsverbesserung und -Verlängerung gefunden worden.
Bei einer weiteren Ausführungsform wird zusätzlich zur Substanzkombination ein Vasokonstriktor verwendet, vorzugsweise Adrenalin, Noradrenalin oder andere, ähnliche, vorzugsweise alpha-adrenerge Vasokonstriktoren. Mit Adrenalin kann die Gesamtdosis der Wirksubstanzen um zirka den Faktor 1,5 - 5 gesteigert werden, da so die systemische Wirkung durch die verminderte Resorption reduziert wird. Die Adrenalinkonzentration kann 1:10'000 bis 1:80'000 bis 1:200'000 betragen. Die Gesamtdosis an Adrenalin liegt bei < 0,25 mg. Eine 50 ml Lösung von 1:200'000 Adrenalin enthält 0,25 mg Adrenalin.
Bei einer weiteren Ausführungsform der Erfindung wird zusätzlich zur Substanzkombination eine antiphlogistisch wirkende Substanz verwendet, z.B. nichtsteroidale Antirheumatika wie COX-2 Hemmer, Acetylsalycylsäure, u.s.w.
Bei einer weiteren Ausführungsform wird zusätzlich zur Substanzkombination oder einer der Komponenten davon ein Lokalanästhetikum verwendet um:
a) nur den Stichkanal zu analgesieren.
b) die Region der Mittelapplikation zu analgesieren.
c) den auszuschaltenden Nerven zu analgesieren,
d) das Rückenmark zu analgesieren
e) die Oberfläche (Haut oder Mucosa oder Urothel oder Darm) auf welche die Substanz(en) aufgetragen werden sollen zu analgesieren.
Das Lokalanästhetikum oder Bestandteile der oder die gesamte Substanzkombination kann dabei sofort freigesetzt werden oder in geeigneter galenischer Form verzögert freigesetzt werden.
Bei einer weiteren Ausführungsform wird zusätzlich zur Substanzkombination ein Steroid verwendet, um beim allfälligen Auftreten einer entzündlichen Reaktion diese zu kontrollieren. Ausserdem kann man damit eine eher kausale Behandlung von schmerzhaften, entzündlichen Gelenkserkrankungen hinzufügen, welche die symptomatische, neurolytische Therapie unterstützt. Als besonders geeignet hat sich Betamethason erwiesen; z.B. 5 mg Betamethason als Diproprionat (kristalline Suspension) und 2 mg Betamethason als Dinatriumphosphat (Lösung in 1 ml, kann der total zu injizierenden Menge beigefügt werden). Diese Lösung ist äquivalent zu 45/23 mg Prednison/Prednisolon.
Bei einer weiteren Ausführungsform wird zusätzlich zur Substanzkombination Glyzerin als Lösungsmittel verwendet. Glyzerin besitzt ebenfalls neurotoxische Eigenschaften (insbesondere aber wenn es intraneural gespritzt wird). Im Weiteren besitzt Glyzerin eine Schmierfähigkeit für das Gelenk, so dass auch eine physikalische Wirkung auftritt. Die Konzentration an Glyzerin beträgt vorzugsweise zwischen 10 und 95 %.
Bei einer weiteren Ausführungsform wird zusätzlich zur Substanzkombination Calcium Ca²⁺ oder vergleichbare Ionen in einer Konzentration höher als physiologisch vorhanden, im Lösungsmittel verwendet und gleichzeitig oder verzögert freigesetzt. Calcium ist für die Wirkung der Vanilloidrezeptoragonisten notwendig und verbessert deren Wirkung wenn in überphysiologischer Konzentration vorhanden. Die Konzentration an Calcium beträgt vorzugsweise >2mMol, insbesondere > 4mMol.
Bei einer weiteren Ausführungsform wird ein am Wirkort ein veränderter pH erzeugt, vorzugsweise durch Mischen des Vanilloidrezeptoragonisten mit einem geeigneten gepufferten Medium. Bei einer Verschiebung des pH kann ein verändertes Wirkprofil erzeugt werden. Bei einem pH kleiner als 7,4 ist die Wirkung des Vanilloidrezeptoragonisten verstärkt, bei einem pH grösser als 7,4 ist die Schmerzhaftigkeit der Injektion deutlich reduziert.
Bei einer weiteren Ausführungsform ist daher mittels geeigneten Puffermedien, welche auch verzögert freigesetzt werden können, durch Mikroenkapsulierung oder in fester Form, z.B. Pulver oder als Implantate wie Knochenersatzmaterial, der pH zuerst bei Applikation oder Injektion höher als 7,4 eingestellt und sinkt danach, vorzugsweise innerhalb von Minuten bis Stunden unter 7,4 ab. Als Lösungsmedium kann anstelle von Glyzerin auch Wasser, Kochsalzlösung, Sodiumiothalamate, lophendylat, Ricin, Poly-Ethlenglycol oder Propylenglycol verwendet werden. Der Vorteil von Glyzerin als Lösungsmittel ist, das dieses hyperbar und an sich auch schon etwas neurotoxisch ist.

Einige Stoffe haben sich als wirkungsverstärkend für die Substanzkombination erwiesen, so z.B. Kalzium (insbesondere in einer Konzentration > 2 mM, vorzugsweise > 4 mM, Magnesium, Antioxidantien, Preservative und Excipienten, insbesondere Natriumbisulfit (> 0,2 %), NaHSO₃, Ammonium-Verbindungen, wie Ammoniumsulfat (NH₄)₂SO₄, 2 - 10 (-30%), Polysorbat 80 (PS80) 0,025 mg/ml.
Die Substanzkombination ist bevorzugt in einem körperverträglichen Lösungsmittel gelöst und wird zweckmässigerweise in einer Volumenmenge injiziert, welche dem verfügbaren Platz im zu behandelnden Gelenk entspricht, so dass dieses sich leicht bis prall füllt. Damit wird der Vorteil einer optimalen lokalen Verteilung der Substanzkombination erreicht. Es ist aber auch möglich weniger Flüssigkeit zu injizieren, dann muss aber das Gelenk gut bewegt werden zur besseren Verteilung der Substanzkombination.
Das in den intrakapsulären Bereich zu injizierende Flüssigkeitsvolumen kann von 0,1 bis 150 ml betragen. Für ein Fingergelenk genügen etwa max. 1ml, für das Schultergelenk max. 10 ml, für das Kniegelenk max. etwa 30 - 50 ml, bevorzugt aber nicht über 2ml.
Die Dosierung der Substanzkombination hängt von der Lokalisation und Indikation ab.

### Systemische Anwendung

Bei dieser Anwendung der Substanzkombination oder Teilen davon wird per oral zusätzlich ein Antiemetikum verwendet.
Bei einer bevorzugten Anwendung der Substanzkombination wird bei der systemischen Anwendung per oral zusätzlich eine magenschonende Substanz verwendet, z.B. ein Protonenpumpenhemmer.

### Topische Anwendung

Bei einer weiteren Ausführungsform wird die erfindungsgemässe Mischung mit einem geeigneten lokalen Patch oder Pflaster appliziert,
Bei einer weiteren Ausführungsform wird die erfindungsgemässe Mischung als Gel, Salbe, Creme, Tinktur oder ähnlichem, fakultativ mit einer permeationsfördernden Substanz, beispielsweise Ethoxyethylendiglycol, gereinigtes Phosphatidylcholin Propyleneglycoldipelargonate (DPPG) oder mit glycolysierten, ethoxylierten Glyceriden zusammen appliziert.
Bei einer weiteren bevorzugten Ausführungsform der Erfindung werden ein Teil oder beide Teile der Substanzkombination mittels lontophorese oder Mikroinjektion (Luft oder hydraulisch oder als Puder) oder ähnlichen Verfahren mit geeigneten üblichen Medien durch die Haut oder Schleimhaut transportiert.

### Regionale Anwendung:

Bei einer weiteren Anwendungsform wird das Rückenmark oder ein peripherer Nerv oder Nervenplexus mit der Substanzkombination oder einem Teil davon zur Behandlung von Schmerzen im Innervationsgebiet oder lokal behandelt.

### Intra/postoperative Anwendung

Bei einer weiteren Anwendung wird das Operationsgebiet oder Teile davon oder eine endoskopisch /arthroskopisch explorierte Wunde, mit der Substanzkombination gespült, beträufelt, diese als Pulver, Paste, in Wachs, oder Gel aufgetragen oder in ähnlicher Form lokal deponiert. So werden die postoperativen Schmerzen reduziert oder verhindert.

### Besondere Ausführungsformen:

Bei einer weiteren Ausführungsform ist der Vanilloidrezeptoragonist ein solcher für den Vanilloid-Rezeptor Typ 1 (TRPV1). Dadurch wird eine selektive Schädigung von Schmerzfasern erzielt.

Bei einer weiteren Ausführungsform ist der Vanilloidrezeptoragonist aus folgenden Substanzen ausgewählt: Resinifera-Verbindungen, wovon insbesondere das Resiniferatoxin (RTX), Olvanil, Capsiate, Civamide, SDZ-249-665, DA-5016, Arvanil, Scutigeral, Isovelleral, Phorbol 12,13-didecanoate 20 homovanillat, Phorbol 12,13-dinonanoate 20-homovanillate, Tinyatoxin und vergleichbare Substanzen, sowie Analoga, Derivate und Salze der vorstehend genannten Verbindungen.

Bei einer weiteren Ausführungsform ist der Vanilloidrezeptoragonist ein Vanilloid, insbesondere aus der Gruppe der trans-8-Methyl-N-vanillyl-6-nonenamide, N-vanillyl-nonanamide, Beta-aminoethyl-substituirte Phenyl-Alkanamide, Methylene substituierteN-Phenylmethyl-Alkanamide, N-[(substituirte-Phenyl)methyl]-cis-monoungesättigte Alkenamide, Beta-Aminoethyl-substituuirte Phenyl-Verbindungen, N-[(substituierte-Phenyl)methyl]doppelt-ungesättigte Amide, N-Oleoyldopamin, ein Capsaicin-Analogon z.B. Trans-Capsaicin, Dihydrocapsaicin, Cis-Capsaicin ist, sowie Analoga, Derivate und Salze der vorstehend genannten Verbindungen. Durch diese Substanzen wird eine selektive Schädigung von Schmerzfasern erzielt.

Bei einer weiteren Ausführungsform enthält die Mischung zusätzlich ein Lokalanästhetikum. Dadurch entstehen weniger Schmerzen bei der Injektion. In geeigneter hoher Konzentration sind die Lokalanästhetika zusätzlich selbst neurotoxisch und unterstützen den gewünschten Effekt.

Bei einer weiteren Ausführungsform enthält die Mischung zusätzlich ein Röntgenkontrastmittel, vorzugsweise in Form von gadoliniumhaltigen, jodhaltigen oder bariumhaltigen Substanzen. Dadurch kann die Verteilung im Körper genau dokumentiert werden.

Bei einer weiteren Ausführungsform enthält die Mischung zusätzlich ein Steroid. Durch diese Mischung können Entzündungsreaktionen unterdrückt werden und ein synergistischer Effekt bezüglich Gelenkschmerz erzielt werden.
Bei einer weiteren Ausführungsform enthält die Mischung zusätzlich einen Vasokonstriktor, vorzugsweise Adrenalin, Noradrenalin, Phenylephrin oder Ornipressin. Dadurch kann eine geringere systemische Verteilung und damit eine bessere systemische Verträglichkeit und bessere lokale Wirksamkeit erzielt werden.
Bei einer weiteren Ausführungsform ist die Mischung in einem körperverträglichen Lösungsmittel gelöst, vorzugsweise einem pharmakologisch akzeptablem Vehikel, insbesondere aus der Gruppe Natriumchlorid-Injektionslösung, Ringiers Injektionslösung, isotonische Dextrose, steriles Wasser, Dextroselösung, Laktierte Ringers Injektionslösung oder Mischungen davon. Dies ist essentiell falls die Substanzmischung injiziert oder die Körperregion damit gespült werden soll.
Bei einer weiteren Ausführungsform enthält die Mischung zusätzlich einen Permeationsförderer, vorzugsweise Dimethylsulfoxid, Ethoxyethylendiglycol, Ethanol, Phosphatidylcholine, Propylenglycol dipelargonate (DPPG), oder glycolysierte ethoxylierte Glyceride. Dies ist insbesondere bei topischer Anwendung auf der Haut, aber auch zur besseren Permeation im Gewebe und durch Schleimhäute von Vorteil. Bei einer weiteren Ausführungsform enthält die Mischung zusätzlich ein Kalziumsalz, wodurch die Wirksamkeit des Vanilloidrezeptoragonisten verbessert wird, da die Toxizität teilweise auf einer Erhöhung des intrazellulären Ca²⁺ Spiegels beruht. Die Kalziumionenkonzentration ist zweckmässigerweise grösser als 2mMol, vorzugsweise grösser als 4mMol. Dadurch wird die Wirksamkeit des Vanilloidrezeptoragonisten verbessert, da die Toxizität teilweise auf einer Erhöhung des intrazellulären Ca²⁺ Spiegels beruht. Derselbe Effekt kann auch erzielt werden, wenn selektiv der Natrium-Spiegel in der Lösung erhöht ist oder wenn die gesamte lonenkonzentration im Lösungsmittel oder Teile davon höher als physiologisch konzentriert sind.

Bei einer weiteren Ausführungsform enthält die Mischung zusätzlich ein Glycosaminoglycan (Chondroitinsulfat), seine Derivate oder Salze. Das Glycosaminoglycan macht zweckmässigerweise 0,5% -10 %, vorzugsweise 1,0 %- 3,0 % der Gesamtmischung aus. Durch die chondroprotektive Wirkung des Glycosaminoglycans wird das brennende Gefühl bei der Injektion unterdrückt und das Gelenk geschont. Eine partielle Bindung des Vanilloidrezeptoragonisten an das Glycosaminoglycan kann eine verlangsamte Abgabe über einen verlängerten Zeitraum bewirken.
Bei einer weiteren Ausführungsform enthält die Mischung zusätzlich eine Hyaluronsäure, ihre Derivate oder Salze. Die Hyaluronsäure macht zweckmässigerweise 0,1 % - 10%, vorzugsweise 0,5% - 3% der Gesamtmischung aus.

Bei einer weiteren Ausführungsform ist die Mischung in einer Pufferlösung mit einem pH-Wert höher als 7,6 , vorzugsweise höher als 8,5 aufgelöst. Dadurch wird die brennende Schmerzhaftigkeit bei der Injektion verringert, da so die Rezeptorlonenkanäle später geöffnet werden.
Bei einer anderen Ausführungsform ist die Mischung in einer Pufferlösung mit einem pH-Wert tiefer als 7,2, vorzugsweise tiefer als 6,0 aufgelöst. Dadurch wird die Wirkung des Vanilloidrezeptoragonisten vergrössert, da sich so die Rezeptor-lonen-Kanäle leichter und früher öffnen.

Bei einer weiteren Ausführungsform ist die Mischung in einer geeigneten galenischen Zubereitung formuliert, welche eine verzögerte Freisetzung der Mischung gestattet. Dadurch kann die Wirkung mit weniger Nebenwirkungen optimiert werden.

Bei einer weiteren Ausführungsform enthält die Mischung eine Kombination von mehreren nervenregenerationshemmenden Substanz enthält. Dadurch erfolgt die Regenerationshemmung noch effizienter und mit weniger Nebenwirkungen als nur mit einer Substanz allein.

Bei einer weiteren Ausführungsform enthält die Mischung eine Kombination von mehreren Vanilloidrezeptoragonisten. Da nicht alle Vanilloirezeptoragonisten auf gleiche Weise an genau denselben Rezeptoren andocken oder diese beeinflussen und nicht dasselbe Nebenwirkungsprofil haben, können Mischungen von solchen Vanilloiden vorteilhafte Synergien entwickeln.

Die erfindungsgemässen Mischungen können zur Herstellung eines Mittels zur Behandlung von Sensationen verwendet werden, welche von Nerven weitergeleitet werden welche Vanilloidrezeptoren tragen. Vanilloidrezeptoragonisten schädigen solche Nerven hoch selektiv.

Bei einer Variante kann das erfindungsgemässe Mittel zur Herstellung eines Mittels zur lokalen Behandlung von Sensationen verwendet werden, welche von Nerven weitergeleitet werden, welche Vanilloidrezeptoren tragen. Vanilloidrezeptoragonisten schädigen solche Nerven hoch selektiv.

Das erfindungsgemässe Mittel ist zur Behandlung folgender Indikationen vorgesehen:
a) Wundschmerzen nach OP in Form einer Spüllösung zur intraoperativen Anwendung bei einer offenen oder arthroskopischen oder endoskopischen Operation, inklusive Liposuction;
b) Gelenkschmerzen durch intraarticuläre Injektion bei
   Arthrose
   Rheumatoider Arthritis
   Infektiöser Arthritis
   Chondrocalzinose
   Ligamentären Schäden
   Meniskusläsion
   Knorpelschaden
   Synovitis
   Arthrofibrose
   Morbus Sudeck
   Nekrose der Gelenkanteile
   Neuropathischen Gelenkschmerzen
c) Knochenschmerzen nach Knochen-Operation durch Auftragung auf den Knochen, nach Beckenkammosteotomie oder Hallux-Valgus Korrektur;
d) Knochenschmerzen durch Injektion in den Knochen bei Femurkopfnekrose in den Femurkopf oder in den Wirbelkörper bei Osteochondrose;
e) Gelenksteife, insbesondere bei Arthrofibrose oder Frozen shoulder;
f) Muskelschmerzen, durch intramuskuläre Injektion, insbesondere bei Muskelfaserriss, Muskelkater oder spastischen Erkrankungen;
g) schmerzhaften Meniskus bei Meniskusdegeneration oder Meniskusriss;
h) Behandlung von Rückenschmerzen durch Injektion in die Bandscheibe bei Bandscheibendegeneration oder Bandscheibenriss
i) schmerzhafte Nerven, insbesondere Trigeminusneuralgie,Neurinom, MortonNeurom, Phantomschmerz oder Narbenneurom
j) Zahnschmerzen, insbesondere bei Karies, allen Formen von Zahnschmerz, Vor/bei/nach Zahnextraktion, Vor/bei/nach Zahnimplantat, lokale Anwendung bei Parodontitis, oder lokale Anwendung bei freiliegenden Zahnhälsen;
k) pleuritischen Beschwerden;
l) Darmbeschwerden, insbesondere bei ColitisUlcerosa, M.Crohn, Analfissur oder Hämorrhoiden.

Bei einer besonderen Verwendungsform weist der Vanilloidrezeptoragonist lokal eine Konzentration oder Dosierung auf, welche äquivalent zu folgenden Parametern ist:
a) im Falle von Resiniferatoxin als Vanilloidrezeptoragonist: eine Konzentration von 5nM bis 500µM, vorzugsweise von 100-5000nM oder eine Dosis von 1ng - 15mg/kg Körpergewicht, vorzugsweise von 10ng - 50µg/kgKörpergewicht.
b) Im Fall von Capsaicin als Vanilloidrezeptoragonist: eine Konzentration von 0,05 % - 10% Gewichtsprozent der Gesamtmischung oder eine Dosis von 0,1-200mg/kg Körpergewicht;

Im Falle von verzögerter Freisetzung z.B. in Form von Mikroenkapsulierung ist die Konzentration in den Kapseln oder in der zu applizierenden Mischung entsprechend höher um die gewünschte lokale Konzentration zu erreichen.

Bei einer besonderen Verwendungsform wird die nervenregenerationshemmende Substanz bei intra-artikulärer Applikation in einer Dosierung von 0,0001mg - 50mg eingesetzt. Die Dosierung beträgt vorzugsweise 0,001mg - 1mg.

Bei einer anderen Verwendungsform wird die nervenregenerationshemmende Substanz bei Applikation per os in einer oder in wiederholten Dosierungen von initial 0,001 - 600mg eingesetzt.

Bei einer besonderen Verwendungsform wird das Mittel in einem geeigneten körperverträglichen Lösungsmittel lokal in die schmerzbefallene Gewebestruktur des Patienten injiziert oder lokal auf die Operationswunde geträufelt, an einen peripheren Nerven oder Ganglion appliziert oder geeignet transcutan appliziert.

Das erfindungsgemässe Mittel kann für ein Verfahren zur Behandlung von Gelenkschmerzen verwendet werden, indem es in den intrakapsulären Bereich oder in den Gelenkschmierbeutel eines von Schmerzen betroffenen Gelenkes lokal injiziert wird. Zweckmässigerweise wird das Mittel in einem körperverträglichen Lösungsmittel gelöst und davon vorzugsweise ein Flüssigkeitsvolumen von 0,1 bis 150 ml in den intrakapsulären Bereich oder in den Gelenkschmierbeutel des von Schmerzen betroffenen Gelenkes lokal injiziert. Dadurch werden die nociceptiven Nervenfasern für mindestens 14 Tage, vorzugsweise mindestens 8 Wochen schmerzunempfindlich gemacht. Das Mittel wird zweckmässigerweise in einer solchen Konzentration verwendet, dass eine Neurolyse auftritt.

Das Mittel kann lokal, regional, systemisch (intravenös, peroral, subcutan, intramuskulär usw.) oder topisch auf der Haut oder Schleimhäuten angewendet werden.

Vorzugsweise werden der Vanilloidrezeptoragonist und die nervenregenerations-hemmende Substanz simultan angewendet. Dadurch kann eine gute lokale Kontrolle und wenig Nebenwirkungen erzielt werden. Insbesondere wird eine gute örtlich und zeitlich synergistische Wirkung beider Substanzen erzielt.
Alternativ kann auch zuerst der Vanilloidrezeptoragonist und danach die nervenregenerationshemmende Substanz angewendet werden. Dadurch kann bei manchen Situationen die vulnerable Phase der Nervenregeneration besser genutzt werden und eine effizientere Wirkung erzielt werden, als bei simultaner Anwendung. Alternativ kann auch zuerst die nervenregenerationshemmende Substanz und danach der Vanilloidrezeptoragonist angewendet werden. Dadurch kann bei manchen Situationen die vulnerable Phase der Nervenregeneration besser genutzt werden und eine effizientere Wirkung erzielt werden.
Der Vanilloidrezeptoragonist und/oder die nervenregenerationshemmende Substanz können auch repetitiv angewendet werden. Dadurch kann bei manchen Situationen die vulnerable Phase der Nervenregeneration besser genutzt werden und eine effizientere Wirkung erzielt werden.
Bei einer besonderen Verwendungsform kann der Vanilloidrezeptoragonist und/oder die nervenregenerationshemmende Substanz mit verzögerter Freisetzung angewendet werden. Dadurch kann bei manchen Situationen die vulnerable Phase der Nervenregeneration besser genutzt werden und eine effizientere Wirkung erzielt werden. Es können so die Nebenwirkungen lokal und systemisch beider Substanzen entscheidend reduziert werden.
Es hat sich als vorteilhaft erwiesen, wenn das zu behandelnde Gelenk vor Applikation des Mittels zur Verminderung von Schmerzen gekühlt wird. Dadurch entstehen bei der Injektion oder Applikation des Substanzgemisches weniger Schmerzen, da die Vanilloid-empfindlichen lonenkanäle sich bei tieferer Temperatur weniger schnell öffnen.
Bei der Anwendung des erfindungsgemässen Mittels kann noch zusätzlich ein oder mehrere Lokalanästhetika verwendet werden, entweder gleichzeitig mit dem Mittel oder vor Anwendung des Mittels. Dadurch wird erreicht, dass die Injektion oder Applikation nicht schmerzhaft ist. Das Lokalanästhetikum kann am selben Ort wie das Mittel oder entfernt davon injiziert werden. Dadurch können die lokalen Schmerzen bei der Injektion verringert werden.
Im Folgenden wird die Erfindung anhand zahlreicher Beispiele näher ausgeführt.

### Beispiel 1:

Der Therapeut brachte unter fakultativer, simultaner (Bildwandler, CT, Sonographie, MRI, Arthroskopie etc.) oder nachträglicher (Röntgen, CT, MRI, Sonographie, etc.) bildgebender Kontrolle eine Spritzennadel in den Gelenksraum eines Kniegelenkes und injizierte 9 ml einer Lösung von 500nM (ca.0.00003mg) Resiniferatoxin mit 0.03mg Vincristin in den intrakapsulären Raum. Der Patient verspürte bereits wenige Tage nach dem Eingriff deutliche Linderung seiner Beschwerden. Diese hielt für über 6 Monate an.

### Beispiel 2:

Der Therapeut brachte unter fakultativer, simultaner (Bildwandler, CT, Sonographie, MRI, Arthroskopie etc.) oder nachträglicher (Röntgen, CT, MRI, Sonographie, etc.) bildgebender Kontrolle eine Spritzennadel in den Gelenksraum eines Kniegelenkes und injizierte 9 ml einer Lösung von 500nM (ca.0.00003mg) Resiniferatoxin mit 0.03mg Vincristin und 1 % (ca. 90mg) Hyaluronsäure in den intrakapsulären Raum. Der Patient verspürte bereits wenige Tage nach dem Eingriff deutliche Linderung seiner Beschwerden. Diese hielt für über 6 Monate an.

### Beispiel 3:

Der Therapeut legte einen dünnen Infusionskatheter analog zu einem Epiduralkatheter in das betroffene Gelenk ein und injizierte mit einem Perfusor eine Mischung von 500nM (ca. 0.0001mg) Resiniferatoxin mit 0,3mg Vinorelbin in das betroffene Gelenk mit einer Rate von 1-10 ml/h während 12 h. Fakultativ legte er noch einen Abflusskatheter ein mit einem fakultativ definierten Abflusswiderstand (z.B. 20 mm Hg), um einen Flüssigkeitsumsatz zu erreichen. Mit dieser Methode erreichte der Therapeut eine gleichmässige Infiltration des schmerzhaften Gelenkes, ohne grosse Konzentrationsspitzen. Ausserdem konnte so die Einwirkzeit besser definiert werden. Bei einer nachfolgenden Arthroskopie nach 1, 2, 7, 14 und 28 d konnte gezeigt werden, dass nur sehr wenig entzündliches Gewebe vorhanden war. Der Patient verspürte bereits 12 Stunden nach dem Eingriff deutliche Linderung seiner Beschwerden. Diese hielt für über 1 Jahr an.

### Beispiel 4:

Nach Implantation einer Kniegelenkprothese injizierte der Therapeut 9 ml einer Mischung von 500nM (ca. 0,0001 mg) Resiniferatoxin mit 0,3mg Vincristin in die wieder verschlossene Gelenkskapsel und das Operationsgebiet. Dadurch konnten die postoperativen Schmerzen minimiert werden.

### Beispiel 5:

Nach Implantation einer Hüftgelenkprothese injizierte der Therapeut 30 ml einer Mischung von 500nM (ca. 0,00003mg) Resiniferatoxin mit 0,01mg Vincristin in den periprothetischen Bereich ohne Kapsel. Dadurch konnten die postoperativen Schmerzen minimiert werden.

### Beispiel 6:

Bei einem Patienten mit schmerzhafter septischer Lockerung einer Hüfttotalendoprothese wurde eine Lösung von 2µM (ca. 0,0004mg) Resiniferatoxin mit 1.5mg Dicoumarol und 0.1mg Taxol in die (Neo)-Kapsel um die Prothese gespritzt werden, was dazu führte, dass der Patient darauf eine dauerhafte (über ein Jahr) Linderung der Schmerzen innerhalb von wenigen (6-12) Stunden erfuhr. Ausserdem wurde die Infektion um die Prothese durch die Diffusion des Schmerzmittels (welche ebenfalls antiseptisch wirkte) entlang des Prothesenschaftes und um die Pfanne stark eingedämmt und in einigen Fällen sogar komplett eliminiert werden. Fakultativ kann diese Behandlung mit systemisch verabreichten Antibiotika (z.B. mit Rifampicin 450 mg, Ciprofloxacin 750 mg) unterstützt werden. Radiologisch konnte eine Konsolidierung der Knochensubstanz um die Prothese gezeigt werden.

### Beispiel 7:

Der Therapeut brachte unter fakultativer, simultaner (Bildwandler, CT, Sonographie, MRI, Arthroskopie etc.) oder nachträglicher (Röntgen, CT, MRI, Sonographie, etc.) bildgebender Kontrolle eine Spritzennadel in den Gelenksraum eines Kniegelenkes nach vorheriger lokaler Analgesie mit 5ml Lidocain 2% und injizierte 90 ml einer Lösung von 0,01mg Olvanil mit 0,03mg Vincristin in physiologischer Kochsalzlösung in den intrakapsulären Raum. Der Patient verspürte bereits wenige Minuten nach dem Eingriff deutliche Linderung seiner Beschwerden. Diese hielt für über 6 Monate an.

### Beispiel 8:

Der Therapeut brachte unter fakultativer, simultaner (Bildwandler, CT, Sonographie, MRI, Arthroskopie etc.) oder nachträglicher (Röntgen, CT, MRI, Sonographie, etc.) bildgebender Kontrolle nach fakultativer lokaler Anästhesie eine Spritzennadel oder einen Katheter in die Blase des Patienten und injizierte 100ml einer Lösung von 500nM Resiniferatoxin mit 0,03mg Vincristin, fakultativ mit 10% Ethanol. Nach 30 Minuten Einwirkzeit wurde die Lösung wieder abgesogen.
Der Patient verspürte bereits Minuten nach dem Eingriff deutliche Linderung seiner Beschwerden. Diese hielt für über 6 Monate an.

### Beispiel 9:

Bei einem Patienten mit schmerzhafter Kapsulitis von Gelenken (z. B. Frozen shoulder oder Arthrofibrose des Kniegelenkes) wurde die eine Mischung von 10ml einer Lösung von 500nM (ca. 0,0001mg) Resiniferatoxin mit 0,3mg Colchizin in physiologischer Kochsalzlösung in das Gelenk injiziert. Wiederum konnte die Verteilung der Substanz, bei Zusatz der entsprechenden Kontrastmittel bildgebend kontrolliert werden. Fakultativ wurde eine antiphlogistisch wirksame Substanz beigemischt. Wenige Minuten nach der Injektion liessen die Schmerzen dauerhaft nach, so dass der Patient mit Physiotherapie die durch die Kapsulitis verlorene Beweglichkeit wiedergewann. Bei dieser Anwendung ist manchmal lediglich eine vorübergehende Analgesie (2-3 Wochen) gewünscht, weshalb hier die Konzentration der neurotoxischen Substanz eher tief gehalten wurde

### Beispiel 10:

Der Therapeut injizierte 0,9 ml einer Lösung bestehend aus 500nM (ca. 0,00001mg) Resiniferatoxin mit 0,03mg Vincristin und fakultativ 1 % Hyaluronsäure und/oder ein Lokalanästhetikum sowie 5 % Kontrastmittel in physiologischer Kochsalzlösung als Lösungsmittel in ein schmerzhaftes, arthrotisches Fingergelenk. Nach circa 15 Minuten verschwanden die Beschwerden des Patienten für mehrere Monate. Die korrekte Position der Injektionsnadel konnte mittels des Kontrastmittels dokumentiert werden.

## Patentansprüche

1. Mischung eines Vanilloidrezeptoragonisten mit einer nervenregenerationshemmenden Substanz, welche aus folgenden Substanzgruppen ausgewählt ist:
a) Vinca-Alkaloide, vorzugsweise das Vincristin, Vincristin-Sulfat, Vinorelbin oder Vinflunin;
b) Colchizine und Colchizin-artige Substanzen;
c) Coumarine oder Dicoumarole.

2. Mischung nach Anspruch 1, **dadurch gekennzeichnet, dass** der Vanilloidrezeptoragonist ein solcher für den Vanilloidrezeptor Typ 1 (TRPV1) ist.

3. Mischung nach Anspruch 1 oder 2, **dadurch gekennzeichnet, dass** der Vanilloidrezeptoragonist aus folgenden Substanzen ausgewählt ist: Resinifera-Verbindungen, wovon insbesondere das Resiniferatoxin (RTX), Olvanil, Capsiate, Civamide, SDZ-249-665, DA-5016, Arvanil, Scutigeral, Isovelleral, Phorbol 12,13-didecanoate 20 homovanillat, Phorbol 12,13-dinonanoate 20-homovanillate, Tinyatoxin und vergleichbare Substanzen, sowie strukturelle Analoga, Derivate und Salze der vorstehend genannten Verbindungen.

4. Mischung nach einem der Ansprüche 1 bis 3, **dadurch gekennzeichnet, dass** der Vanilloidrezeptoragonist, ein Vanilloid ist, insbesondere aus der Gruppe der trans-8-Methyl-N-vanillyl-6-nonenamide, N-vanillyl-nonanamide, Beta-aminoethyl-substituierte Phenyl-Alkanamide, Methylene substituierte-N-Phenylmethyl-Alkanamide, N-[(substituierte-Phenyl)methyl]-cis-monoungesättigte Alkenamide, Beta-Aminoethyl-substituierte Phenyl-Verbindungen, N-[(substituierte-Phenyl)methyl]doppelt-ungesättigte Amide, N-Oleoyldopamin, Trans-Capsaicin, Dihydrocapsaicin oder Cis-Capsaicin, sowie Derivate und Salze der vorstehend genannten Verbindungen.

5. Mischung nach einem der Ansprüche 1 bis 4, **dadurch gekennzeichnet, dass** sie zusätzlich ein Lokalanästhetikum enthält.

6. Mischung nach einem der Ansprüche 1 bis 5, **dadurch gekennzeichnet, dass** sie zusätzlich ein Röntgenkontrastmittel enthält, vorzugsweise in Form von gadoliniumhaltige, jodhaltige oder bariumhaltige Substanzen.

7. Mischung nach einem der Ansprüche 1 bis 6, **dadurch gekennzeichnet, dass** sie zusätzlich ein Steroid enthält.

8. Mischung nach einem der Ansprüche 1 bis 7, **dadurch gekennzeichnet, dass** sie zusätzlich einen Vasokonstriktor enthält, vorzugsweise Adrenalin, Noradrenalin, Phenylephrin oder Ornipressin.

9. Mischung nach einem der Ansprüche 1 bis 8, **dadurch gekennzeichnet, dass** sie in einem körperverträglichen Lösungsmittel gelöst ist, vorzugsweise einem pharmakologisch akzeptablem Vehikel, insbesondere aus der Gruppe Natriumchlorid-Injektionslösung, Ringiers Injektionslösung, isotonische Dextrose, steriles Wasser Dextroselösung, Laktierte Ringers Injektionslösung, destilliertes Wasser oder Mischungen davon.

10. Mischung nach einem der Ansprüche 1 bis 9, **dadurch gekennzeichnet, dass** sie zusätzlich einen Permeationsförderer, vorzugsweise Dimethylsulfoxid, Ethoxyethylendiglycol, Ethanol, Phosphatidylcholine, Propylenglycol dipelargonate (DPPG), oder glycolysierte ethoxylierte Glyceride enthält.

11. Mischung nach einem der Ansprüche 1 bis 10, **dadurch gekennzeichnet, dass** sie zusätzlich ein Kalziumsalz enthält.

12. Mischung nach Anspruch 11, **dadurch gekennzeichnet, dass** die Kalziumionenkonzentration grösser als 2mMol, vorzugsweise grösser als 4mMol beträgt.

13. Mischung nach Anspruch 11 oder 12, **dadurch gekennzeichnet, dass** die im Lösungsmedium gelösten Salze und Ionen höher als physiologisch normal (z.B in Ringer Lactat Lösung) konzentriert sind.

14. Mischung nach einem der Ansprüche 1 bis 13, **dadurch gekennzeichnet, dass** sie zusätzlich ein Glycosaminoglycan (Chondroitinsulfat), seine Derivate oder Salze enthält.

15. Mischung nach Anspruch 14, **dadurch gekennzeichnet, dass** das Glycosaminoglycan 0,5% - 10 %, vorzugsweise 1,0 %- 3,0 % der Gesamtmischung ausmacht.

16. Mischung nach einem der Ansprüche 1 bis 15, **dadurch gekennzeichnet, dass** sie zusätzlich eine Hyaluronsäure, ihre Derivate oder Salze enthält.

17. Mischung nach Anspruch 16, **dadurch gekennzeichnet, dass** die Hyaluronsäure 0,1% - 10%, vorzugsweise 0,5% - 3% der Gesamtmischung ausmacht.

18. Mischung nach einem der Ansprüche 1 bis 17, **dadurch gekennzeichnet, dass** sie in einer Pufferlösung mit einem pH-Wert höher als 7,6 , vorzugsweise höher als 8,5 aufgelöst ist.

19. Mischung nach einem der Ansprüche 1 bis 18, **dadurch gekennzeichnet, dass** sie in einer Pufferlösung mit einem pH-Wert tiefer als 7,2, vorzugsweise tiefer als 6,5 aufgelöst ist.

20. Mischung nach einem der Ansprüche 1 bis 19, **dadurch gekennzeichnet, dass** sie in einer geeigneten galenischen Zubereitung formuliert ist, welche eine verzögerte Freisetzung der Mischung gestattet.

21. Mischung nach einem der Ansprüche 1 bis 20, **dadurch gekennzeichnet, dass** sie eine Kombination von mehreren nervenregenerationshemmenden Substanzen enthält.

22. Mischung nach einem der Ansprüche 1 bis 21, **dadurch gekennzeichnet, dass** sie eine Kombination von mehreren Vanilloidrezeptoragonisten enthält.

23. Verwendung der Mischung nach einem der Ansprüche 1 bis 22 für die Herstellung eines Mittels zur Behandlung von Sensationen welche von Nerven weitergeleitet werden, welche Vanilloidrezeptoren tragen.

24. Verwendung nach Anspruch 23 für die Herstellung eines Mittels zur lokalen Behandlung von Sensationen, welche von Nerven weitergeleitet werden welche Vanilloidrezeptoren tragen.

25. Verwendung nach Anspruch 23 oder 24, **dadurch gekennzeichnet, dass** das Mittel zur Behandlung folgender Indikationen vorgesehen ist:
a) Wundschmerzen nach OP in Form einer Spüllösung zur intraoperativen Anwendung bei einer offenen oder arthroskopischen oder endoskopischen Operation, inklusive Liposuction;
b) Gelenkschmerzen durch intraarticuläre Injektion bei
Arthrose
Rheumatoider Arthritis
Infektiöser Arthritis
Chondrocalzinose
Ligamentären Schäden
Meniskusläsion
Knorpelschaden
Synovitis
Arthrofibrose
Morbus Sudeck
Nekrose der Gelenkanteile
Neuropathischen Gelenkschmerzen
c) Knochenschmerzen nach Knochen-Operation durch Auftragung auf den Knochen, nach Beckenkammosteotomie oder Hallux-Valgus Korrektur;
d) Knochenschmerzen durch Injektion in den Knochen bei Femurkopfnekrose in den Femurkopf oder in den Wirbelkörper bei Osteochondrose;
e) Gelenksteife, insbesondere bei Arthrofibrose oder Frozen shoulder;
f) Muskelschmerzen, durch intramuskuläre Injektion, insbesondere bei Muskelfaserriss, Muskelkater oder spastischen Erkrankungen;
g) schmerzhaften Meniskus bei Meniskusdegeneration oder Meniskusriss;
h) Behandlung von Rückenschmerzen durch Injektion in die Bandscheibe bei Bandscheibendegeneration oder Bandscheibenriss
i) schmerzhafte Nerven, insbesondere Trigeminusneuralgie,Neurinom, MortonNeurom, Phantomschmerz oder Narbenneurom
j) Zahnschmerzen, insbesondere bei Karies, allen Formen von Zahnschmerz, Vor/bei/nach Zahnextraktion, Vor/bei/nach Zahnimplantat, lokale Anwendung bei Parodontitis, oder lokale Anwendung bei freiliegenden Zahnhälsen;
k) pleuritischen Beschwerden;
l) Darmbeschwerden, insbesondere bei Colitis Ulcerosa, M.Crohn, Analfissur oder Hämorrhoiden.

26. Verwendung nach einem der Ansprüche 23 bis 25, **dadurch gekennzeichnet, dass** der Vanilloidrezeptoragonist lokal eine Konzentration oder Dosierung aufweist, welche äquivalent zu folgenden Parametern ist:
a) im Falle von Resiniferatoxin als Vanilloidrezeptoragonist: eine Konzentration von 5nM bis 500µM, vorzugsweise von 100-5000nM oder eine Dosis von 1ng - 15mg/kg Körpergewicht, vorzugsweise von 10ng - 50µg/kgKörpergewicht;
b) im Fall von Capsaicinoiden als Vanilloidrezeptoragonist: eine Konzentration von 0,05 % -10% Gewichtsprozent der Gesamtmischung oder eine Dosis von 0,1-200mg/kg Körpergewicht.

27. Verwendung nach einem der Ansprüche 23 bis 26, **dadurch gekennzeichnet, dass** die nervenregenerationshemmende Substanz bei intra-artikulärer Applikation in einer Dosierung von 0,0001mg - 50mg eingesetzt wird.

28. Verwendung nach Anspruch 27, **dadurch gekennzeichnet, dass** die Dosierung 0,001mg - 1mg beträgt.

29. Verwendung nach einem der Ansprüche 23 bis 28, **dadurch gekennzeichnet, dass** die nervenregenerationshemmende Substanz bei Applikation per os in einer oder in wiederholten Dosierungen von initial 0,001 - 600mg eingesetzt wird.

30. Verwendung nach einem der Ansprüche 23 bis 29, **dadurch gekennzeichnet, dass** das Mittel in einem geeigneten körperverträglichen Lösungsmittel lokal in die schmerzbefallene Gewebestruktur des Patienten injiziert wird oder lokal auf die Operationswunde geträufelt wird oder an einen peripheren Nerven oder Ganglion appliziert wird oder geeignet transcutan appliziert wird.

31. Zusammensetzung, welche zwei Komponenten umfasst, **dadurch gekennzeichnet, dass**
A) die erste Komponenten einen Vanilloidrezeptoragonisten enthält; und
B) die zweite Komponenten eine nervenregenerations-hemmende Substanz enthält, welche aus den folgenden Substanzgruppen ausgewählt ist:
a) Vinca-Alkaloide, vorzugsweise das Vincristin, Vincristin-Sulfat, Vinorelbin oder Vinflunin;
b) Colchizine und Colchizin-artige Substanzen; sowie
c) Coumarine oder Dicoumarole.

## Claims

1. Mixture of a vanilloid receptor agonist with a substance, which inhibits nerve regeneration and is selected from the following groups of substances:
a) vinca alkaloids, preferably Vincristin, Vincristin sulfate, Vinorelbin or Vinflunin;
b) colchicine and colchicine-like substances;
c) coumarins or dicoumarols.

2. The mixture of claim 1, **characterized in that** the vanilloid receptor agonist is an agonist for the type 1 vanilloid receptor (TRPV1).

3. The mixture of claim 1 or 2, **characterized in that** the vanilloid receptor agonist is selected from the following substances: resinifera compounds, of which, in particular, the resiniferatoxin (RTX), olvanil, capsiate, civamide, SDZ-249-665, DA-5016, arvanil, scutigeral, isovelleral, phorbol 12,13-didecanoate 20 homovanillate, phorbol 12,13-dinonanoates 20-homovanillates, tinyatoxin and comparable substances, as well as analogs, derivatives and salts of the compounds mentioned above.

4. The mixture of one of the claims 1 to 3, **characterized in that** the vanilloid receptor agonist is a vanilloid, particularly from the group of trans-8-methyl-N-vanillyl-6-nonenamides, N-vanillyl-nonanamides, beta-aminoethyl-substituted phenylalkanamides, methylene substituted N-phenylmethylalkanamides, N-((substituted phenyl)methyl)-cis-monounsaturated alkenamides, beta-aminoethyl-substituted phenyl compounds, N-((substituted phenyl)methyl) diunsaturated amides, N-oleoyl dopamine, transcapsaicin, dihydrocapsaicin or cis-capsaicin, as well as derivatives and salts of the aforementioned compounds.

5. The mixture of one of the claims 1 to 4, **characterized in that** it contains additionally a local anesthetic.

6. The mixture of one of the claims 1 to 5, **characterized in that** it contains additionally an x-ray contrasting agent, preferably in the form of gadolinium-containing, iodine-containing or barium-containing substances.

7. The mixture of one of the claims 1 to 6, **characterized in that** it contains additionally a steroid.

8. The mixture of one of the claims 1 to 7, **characterized in that** it contains additionally a vasoconstrictor, preferably adrenaline, noradrenaline, phenylephrine or ornipressin.

9. The mixture of one of the claims 1 to 8, **characterized in that** it is dissolved in a solvent, which is compatible with the body, preferably a pharmacologically acceptable vehicle, particularly from the group of sodium chloride injection solution, Ringer's injection solution, isotonic dextrose, sterile water dextrose solution, lactated Ringer's injection solution, distilled water or mixtures thereof.

10. The mixture of one of the claims 1 to 9, **characterized in that** it contains additionally a permeation promoter, preferably dimethyl sulfoxide, ethoxylated ethylene diglycol, ethanol, phosphatidyl cholines, propylene glycol dipelargonates (DPPG), or glycosylated ethoxylated glycerides.

11. The mixture of one of the claims 1 to 10, **characterized in that** it additionally contains a calcium salt.

12. The mixture of claim 11, **characterized in that** a calcium ion concentration is greater than 2 mmolar and preferably greater than 4 mmolar.

13. The mixture of claims 11 or 12, **characterized in that** the concentration of salts and islands, dissolved in the solvent, is higher than that in a physiologically normal solution, such as a Ringer lactate solution.

14. The mixture of one of the claims 1 to 13, **characterized in that** it contains additionally a glycosaminoglycan (chondroitin sulfate), its derivatives or salts.

15. The mixture of claim 14, **characterized in that** the glycosaminoglycan constitutes 0.5% to 10% and preferably 1.0% to 3.0% of the total mixture.

16. The mixture of one of the claims 1 to 15, **characterized in that** it contains additionally a hyaluronic acid, its derivatives or salts.

17. The mixture of claim 16, **characterized in that** the hyaluronic acid constitutes 0.1% to 10% and preferably 0.5% to 3% of the total mixture.

18. The mixture of one of the claims 1 to 17, **characterized in that** it is dissolved in a buffer solution having a pH higher than 7.6 and preferably higher than 8.5.

19. The mixture of one of the claims 1 to 18, **characterized in that** it is dissolved in a buffer solution having a pH lower than 7.2 and preferably lower than 6.5.

20. The mixture of one of the claims 1 to 19, **characterized in that** it is formulated in a suitable galenical preparation, which permits the release of the mixture to be retarded.

21. The mixture of one of the claims 1 to 20, **characterized in that** it contains a combination of several substances, which inhibit nerve regeneration.

22. The mixture of one of the claims 1 to 21, **characterized in that** it contains a combination of several vanilloid receptor agonists.

23. Use of the mixture of one of the claims 1 to 22 for producing an agent for the treatment of sensations, which are passed on by nerves carrying vanilloid receptors.

24. The use of claim 23 for producing an agent for the topical treatment of sensations, which are passed on by nerves carrying vanilloid receptors.

25. The use of claims 23 or 24, **characterized in that** the agent is intended to treat the following indications:
a) Wound pain after surgery in the form of a flushing solution for intraoperative application for open or arthroscopic or endoscopic surgery, including liposuction;
b) Joint pain by intraarticular injection in the case of
arthrosis
rheumatoid arthritis
infectious arthritis
chondrocalcinosis
ligamentary damage
meniscus lesion
cartilage damage
synovitis
arthrofibrosis
Sudeck's disease
necrosis of portions of a joint
neuropathic joint pain
c) Bone pain after bone surgery by application on the bone after iliac crest osteotomy or Hallux-Valgus correction
d) Bone pain by injection into the bone in the case of necrosis of the head of the femur or into the body of a vertebra in the case of osteochondrosis;
e) Joint stiffness, especially in the case of arthrofibrosis or a frozen shoulder;
f) Muscle pain due to intramuscular injection, especially if there is a tear in muscle fibers, if there is pain after muscular exertion or in the case of spastic diseases;
g) Painful meniscus if there is degeneration of or a tear in the meniscus;
h) Treatment of back pain by injection into the intervertebral disk in the case of the degeneration of or a tear in the intervertebral disk;
i) Painful nerves, especially trigeminus neuralgia, neurinoma, Morton neurinoma, phantom pain or scar neuroma;
j) Toothache, especially in the case of dental caries, all forms of tooth ache, before, during or after tooth extraction, before, during or after a tooth implant, applied topically in the case of parodontitis, or applied topically in the case of an exposed neck of a tooth;
k) Pleuritic complaints
l) Intestinal complaints, especially in the case of ulcerous colitis, Crohn's disease, anal fissures or hemorrhoids.

26. The use of one of the claims 23 to 25, **characterized in that** the vanilloid receptor agonist locally has a concentration or dosage, which is equivalent to the following parameters:
a) In the case of resiniferatoxin as vanilloid receptor agonists, a concentration of 5 nmolar to 600 µmolar and preferably of 100 to 5000 nmolar or a dose of 1 ng to 15 mg/kg of body weight and preferably of 10 ng to 50 µg/kg of body weight.
b) In the case of capsaicinoids as vanilloid receptor agonist, a concentration of 0.05% to 10% by weight of the total mixture or a dose of 0.1 to 200 mg/kg of body weight.

27. The use of one of the claims 23 to 26, **characterized in that** the substance, inhibiting nerve regeneration, is used at a dosage of 0.0001 mg to 50 mg for intra-articular application.

28. The use of claim 27, **characterized in that** the dosage is 0.001 mg to 1 mg.

29. The use of one of the claims 23 to 28, **characterized in that** the substance, inhibiting regeneration of nerves, is used for application per os in one dosage or in repeated dosages initially of 0.001 to 600 mg.

30. The use of one of the claims 23 to 29, **characterized in that** the agent is injected locally in a suitable solvent, which is compatible with the body, into the pain-affected tissue structure of the patient or administered dropwise topically onto the surgical wound or applied at a peripheral nerve or ganglion or applied suitably transcutaneously.

31. Composition comprising two components, **characterized in that**
A) the first component comprises a vanilloid receptor agonist; and
B) the second component comprises a substance, which inhibits nerve regeneration and which is selected from the following groups of substances:
a) vinca alkaloids, preferably Vincristin, Vincristin sulfate, Vinorelbin or Vinflunin;
b) colchicine and colchicine-like substances;
c) coumarins or dicoumarols.

## Revendications

1. Mélange d'un agoniste des récepteurs vanilloïdes avec une substance inhibant la régénération nerveuse, laquelle est choisie dans le groupe de substances suivant :
a) les vinca-alcaloïdes, de préférence la vincristine, le sulfate de vincristine, la vinorelbine ou la vinflunine ;
b) les colchicines et les substances analogues à la colchicine ;
c) les coumarines ou les dicoumaroles.

2. Mélange selon la revendication 1, **caractérisé en ce que** l'agoniste des récepteurs vanilloïdes fait partie de ceux qui sont spécifiques des récepteurs vanilloïdes de type 1 (TRPV1).

3. Mélange selon la revendication 1 ou 2, **caractérisé en ce que** l'agoniste des récepteurs vanilloïdes est choisi parmi les substances suivantes : composés de résiniféra, parmi lesquels, en particulier, la résinifératoxine (RTX), l'olvanil, le capsiate, le civamide, SDZ-249-665, DA-5016, l'arvanil, le scutigéral, l'isovelléral, le phorbol 12,13-didécanoate 20-homovanillate, le phorbol 12,13-dinonanoate 20-homovanillate, la tinyatoxine et substances analogues, ainsi que les analogues structurels, les dérivés et les sels des composés susmentionnés.

4. Mélange selon l'une quelconque des revendications 1 à 3, **caractérisé en ce que** l'agoniste des récepteurs vanilloïdes est un vanilloïde, issu en particulier du groupe des trans-8-méthyl-N-vanillyl-6-nonenamides, des N-vanillyl-nonanamides, des phényl-alkanamides à substitution bêta-aminoéthyle, des N-phénylméthyl-alkanamides à substitution méthylène, des N-[(phényle substitué)méthyl]-cis-alcénamides mono-insaturés, des composés phényliques à substitution bêta-aminoéthyle, des N-[(phényle substitué)méthyl]-amides di-insaturés, la N-oléoyldopamine, la transcapsaïcine, la dihydrocapsaïcine ou la cis-capsaïcine, ainsi que les dérivés et les sels des composés susmentionnés.

5. Mélange selon l'une quelconque des revendications 1 à 4, **caractérisé en ce qu'**il contient en outre un anesthésiant local.

6. Mélange selon l'une quelconque des revendications 1 à 5, **caractérisé en ce qu'**il contient en outre un agent de contraste radiographique, de préférence sous la forme de substances contenant du gadolinium, de l'iode ou du baryum.

7. Mélange selon l'une quelconque des revendications 1 à 6, **caractérisé en ce qu'**il contient en outre un stéroïde.

8. Mélange selon l'une quelconque des revendications 1 à 7, **caractérisé en ce qu'**il contient en outre un vasoconstricteur, de préférence l'adrénaline, la noradrénaline, la phényléphrine ou l'ornipressine.

9. Mélange selon l'une quelconque des revendications 1 à 8, **caractérisé en ce qu'**il est dissous dans un solvant toléré par le corps, de préférence un véhicule acceptable sur le plan pharmacologique, choisi en particulier dans le groupe comprenant une solution pour préparation injectable de chlorure de sodium, une solution pour préparation injectable de Ringer, la dextrose isotonique, l'eau stérile, une solution de dextrose, une solution pour préparation injectable de Ringer Lactate, l'eau distillée ou les mélanges de celles-ci.

10. Mélange selon l'une quelconque des revendications 1 à 9, **caractérisé en ce que** qu'il contient en outre un agent facilitant la perméation, de préférence le diméthylsulfoxyde, l'éthoxyéthylènediglycol, l'éthanol, la phosphatidylcholine, le dipélargonate de propylèneglycol (DPPG), ou des glycérides glycolysés et éthoxylés.

11. Mélange selon l'une quelconque des revendications 1 à 10, **caractérisé en ce qu'**il contient en outre un sel de calcium.

12. Mélange selon la revendication 11, **caractérisé en ce que** sa concentration en ions calcium est supérieure à 2 mM, de préférence supérieure à 4 mM.

13. Mélange selon la revendication 11 ou 12, **caractérisé en ce que** les sels et ions dissous dans le milieu de dissolution sont présents dans une concentration supérieure à une concentration physiologique normale (par exemple, dans la solution de Ringer Lactate).

14. Mélange selon l'une quelconque des revendications 1 à 13, **caractérisé en ce qu'**il contient en outre un glycosaminoglycane (sulfate de chondroïtine), ses dérivés ou sels.

15. Mélange selon la revendication 14, **caractérisé en ce que** le glycosaminoglycane constitue 0,5% à 10%, de préférence 1,0% à 3,0% du mélange total.

16. Mélange selon l'une quelconque des revendications 1 à 15, **caractérisé en ce qu'**il contient en outre un acide hyaluronique, ses dérivés ou sels.

17. Mélange selon la revendication 16, **caractérisé en ce que** l'acide hyaluronique constitue 0,1% à 10%, de préférence 0,5% à 3% du mélange total.

18. Mélange selon l'une quelconque des revendications 1 à 17, **caractérisé en ce qu'**il est dissous dans une solution tampon ayant un pH supérieur à 7,6, de préférence supérieur à 8,5.

19. Mélange selon l'une quelconque des revendications 1 à 18, **caractérisé en ce qu'**il est dissous dans une solution tampon ayant un pH inférieur à 7,2, de préférence inférieur à 6,5.

20. Mélange selon l'une quelconque des revendications 1 à 19, **caractérisé en ce qu'**il est formulé dans une préparation galénique appropriée, laquelle permet une libération prolongée du mélange.

21. Mélange selon l'une quelconque des revendications 1 à 20, **caractérisé en ce qu'**il contient une combinaison de plusieurs substances inhibant la régénération nerveuse.

22. Mélange selon l'une quelconque des revendications 1 à 21, **caractérisé en ce qu'**il contient une combinaison de plusieurs agonistes des récepteurs vanilloïdes.

23. Utilisation du mélange selon l'une quelconque des revendications 1 à 22 pour la préparation d'un agent pour le traitement des sensations transmises par les nerfs porteurs de récepteurs vanilloïdes.

24. Utilisation selon la revendication 23, pour le traitement local des sensations transmises par les nerfs porteurs de récepteurs vanilloïdes.

25. Utilisation selon la revendication 23 ou 24, **caractérisée en ce que** l'agent est destiné au traitement des indications suivantes :
a) douleurs post-opératoires liées aux plaies, sous la forme d'une solution de rinçage à utilisation intraopératoire lors d'une opération à ciel ouvert ou arthroscopique ou endoscopique, y compris la liposuccion ;
b) douleurs articulaires, par injection intraarticulaire en cas de :
arthrose
polyarthrite rhumatoïde
arthrite infectieuse
chondrocalcinose
lésions ligamentaires
lésion du ménisque
lésions cartilagineuses
synovite
arthrofibrose
maladie de Sudeck
nécrose de parties articulaires
douleurs articulaires neuropathiques
c) douleurs osseuses consécutives à une chirurgie osseuse, par application sur l'os, après une ostéotomie de la crête iliaque ou une correction d'hallux valgus ;
d) douleurs osseuses, par injection dans la tête fémorale dans le cas d'une nécrose de la tête fémorale ou par injection dans le corps vertébral dans le cas d'une ostéochondrose ;
e) raideur articulaire, en particulier dans le cas d'une arthrofibrose ou d'une épaule gelée ;
f) douleurs musculaires, par injection intramusculaire, en particulier dans le cas d'une déchirure musculaire, de courbatures ou de troubles spasmodiques ;
g) douleurs au niveau du ménisque dans le cas d'une dégénérescence ou d'une déchirure du ménisque ;
h) douleurs dorsales, par injection dans le disque intervertébral dans le cas d'une dégénérescence du disque intervertébral ou d'une hernie discale ;
i) névralgies, en particulier névralgie trigéminale, neurinome, névrome de Morton, douleur fantôme ou cicatrice névromateuse ;
j) douleurs dentaires, en particulier dans le cas de caries, de toutes les formes de douleurs dentaires avant/pendant/après une extraction dentaire, avant/pendant/après une implantation dentaire, par application locale dans le cas d'une parodontite, ou par traitement local dans le cas de dénudations du collet dentaire ;
k) pleurésies ;
l) troubles intestinaux, en particulier dans le cas d'une rectocolite hémorragique, de la maladie de Crohn, d'une fissure anale ou d'hémorroïdes.

26. Utilisation selon l'une quelconque des revendications 23 à 25, **caractérisée en ce que** l'agoniste des récepteurs vanilloïdes présente localement une concentration ou un dosage équivalent aux paramètres suivants :
a) dans le cas de la résinifératoxine en tant qu'agoniste des récepteurs vanilloïdes : une concentration de 5 nM à 500 µM, de préférence de 100 nM à 5 000 nM ou une dose de 1 ng à 15 mg/kg de masse corporelle, de préférence de 10 ng à 50 µg/kg de masse corporelle ;
b) dans le cas des capsaicinoïdes en tant qu'agoniste des récepteurs vanilloïdes : une concentration de 0,05% à 10% en poids du mélange total ou une dose de 0,1 à 200 mg/kg de masse corporelle.

27. Utilisation selon l'une quelconque des revendications 23 à 26, **caractérisée en ce que** la substance inhibant la régénération nerveuse est utilisée pour une application intra-articulaire à une dose de 0,0001 mg à 50 mg.

28. Utilisation selon la revendication 27, **caractérisée en ce que** la dose est de 0,001 mg à 1 mg.

29. Utilisation selon l'une quelconque des revendications 23 à 28, **caractérisée en ce que**, lors d'une administration par voie orale, la substance inhibant la régénération nerveuse est administrée en une ou plusieurs doses répétitives, initialement de 0,001 à 600 mg.

30. Utilisation selon l'une quelconque des revendications 23 à 29, **caractérisée en ce que** l'agent dissous dans un solvant toléré par le corps est injecté localement dans la structure tissulaire douloureuse du patient ou instillé goutte à goutte sur la plaie opératoire ou appliqué au niveau d'un nerf périphérique ou d'un ganglion ou administré de manière appropriée par voie transcutanée.

31. Composition comprenant deux composants, **caractérisée en ce que** :
A) le premier composant contient un agoniste des récepteurs vanilloïdes ; et
B) le deuxième composant contient une substance inhibant la régénération nerveuse choisie dans les groupes de substances suivants :
a) les vinca-alcaloïdes, de préférence la vincristine, le sulfate de vincristine, la vinorelbine ou la vinflunine ;
b) les colchicines et les substances analogues à la colchicine ; ainsi que
c) les coumarines ou les dicoumaroles.
